# EUROPÄISCHE PATENTSCHRIFT

(11) **EP 1 946 710 B1**
(45) Veröffentlichungstag und Bekanntmachung des Hinweises auf die Patenterteilung: **03.07.2013**
(21) Anmeldenummer: 07121002.5
(22) Anmeldetag: 19.11.2007
(51) Int. Cl.: A61B 17/88, A61B 19/00, A61B 17/16

(54) **Chirurgisches Instrument zur Implantierung eines Drahtes, vorzugsweise in einen Knochen**
Surgical instrument for implanting a wire, in particular in a bone
Instrument chirurgical destiné à l'implantation d'un fil, en particulier dans un os

(30) Priorität: 06.12.2006 DE 202006018587 U
(43) Veröffentlichungstag der Anmeldung: 23.07.2008
(73) Patentinhaber: Zrinski AG, 78573 Wurmlingen (DE)
(72) Erfinder: Reitzig, Cliff-Georg, 78604, Weilheim-Rietheim (DE); Eckhof, Stephan, 78604, Weilheim-Rietheim (DE); Feldhaus, Thomas, 78532, Tuttlingen (DE)

(56) Entgegenhaltungen:
- WO-A-01/35843
- WO-A-96/28103
- DE-A1-102004 033 633
- DE-U1- 29 900 616
- DE-U1- 29 912 625
- US-A- 3 351 054
- US-A1- 2004 225 308

## Beschreibung

### Technisches Gebiet

Die Erfindung betrifft ein chirurgisches Instrument. Insbesondere betrifft die Erfindung ein chirurgisches Instrument zur Handhabung eines geraden oder gebogenen, chirurgischen Drahtes für die Anwendung bei einem intraoperativen Eingriff.

### Hintergrund der Erfindung

Chirurgische Drähte, beispielsweise Kirschnerdrähte (oder K-Drähte) werden in der Regel zur Fixierung von Knochenfrakturen verwendet. Zur Fixierung wird in einem ersten Schritt jeweils eine Bohrung in dem zu fixierenden Knochenfragment und in dem benachbarten Knochen erstellt. In einem weiteren Schritt wird der Draht durch diese beiden Bohrungen hindurchgeführt und beispielsweise durch Verdrehen oder Verbinden mit weiteren Fixierelementen derart positioniert, dass die Fraktur fixiert ist und so auf diese Weise ein Zusammenwachsen des Knochens wieder möglich ist.

Zur Durchführung dieses Vorgangs sind unterschiedliche Werkzeuge notwendig. Zum einen sind Hilfsmittel vorgesehen, die es ermöglichen, zunächst den Draht an seinem freien Ende zu biegen, damit eine Einführung in eine Bohrung, die in dem Knochen vorgesehen ist, möglich ist. Entsprechende Biegewerkzeuge sind hierfür vorgesehen. Weitere Hilfswerkzeuge dienen dazu, den Draht in die beiden Bohrung einzuführen. Ist der Draht durch beide Bohrungen geführt worden, ist es bekannt, eine Drahtzange zu verwenden, mit dieser das aus dem Knochen herausragende Ende abzubiegen und gegebenenfalls abzuschneiden ist. Dadurch wird der Draht an seinem knochennahen Ende in einer solchen Drahtzange fixiert und beim weiteren Zusammendrücken der Handgriffe entsprechend gebogen (Zuggurtungs-Osteosynthese). Bei der klassischen Zuggurtungs-Osteosynthese wird das aus dem Knochen herausragende Ende des Kirschnerdrahtes dabei auf eine bestimmte Länge gekürzt und danach umgebogen. Durch die Umbiegung und das Hereindrücken des Drahtendes in den Knochen wird das Wandern des Drahtes im Knochen sowie die Rotation des Drahts um seine Längsachse verhindert. Sofern Knochenplatten zusätzlich zur Stützung der Fraktur verwendet werden, sind weitere Instrumente wie Schneidzangen, Biegezangen und ähnliches, zu verwenden.

Die am häufigsten verwendeten Drahtstärken liegen zwischen 0,8 und 2 mm. Die Verwendung der entsprechenden Durchmesser hängt sehr stark von der Beanspruchung der einzelnen Knochenfragmente ab. Stark belastete Bereiche, wie beispielsweise die Hüfte, bedürfen zur Drahtfixierung stärkere Durchmesser, wohingegen bei weniger stark belastete Knochenfragmente entsprechend dünnere Drahtstärken Anwendung finden.

Das Einführen des Drahtes in eine Bohrung, die in einem Knochen vorgesehen ist, erfolgt in der Regel mit handelsüblichen chirurgischen Instrumenten, wie beispielsweise einer Zange. Hierzu greift der Operateur den chirurgischen Draht mit den Klemmbacken der Zange und führt den Draht in die Bohrung ein. Hat er mit den Klemmbacken nahezu die Knochenaussenwahndung erreicht, so ist die Zange zu lösen und der Draht an einer weiteren, von dem Knochen beabstandeten Stelle erneut zu klemmen. Durch Aufbringen der Klemmkraft ist es möglich, dass durch Vorschieben der Zange in Richtung des Knochens der Draht weiter in die Bohrung eingeführt wird.

### Stand der Technik

Zum Einführen des Drahtes, beispielsweise eines Kirschnerdrahtes (K-Draht) sind handelsübliche chirurgische Instrumente bekannt. Sie sind zangenartig ausgebildet und in der Art gestaltet, dass der Draht zwischen zwei Klemmbacken geklemmt wird. Die Klemmbacken sind zangenartig mit Griffelementen verbunden, so dass durch Zusammendrücken der Griffteile, die das Griffelement bilden, eine Klemmkraft auf den Draht ausgeübt wird. Die Klemmbacken selbst sind in der Regel flach ausgebildet, so dass es nicht unbedingt notwendig ist, eine exakte Positionierung der Klemmbacken vorzunehmen, um einen Vortrieb innerhalb der Bohrung in dem Knochen zu bewirken.

DE 102004033633 A (STRYKER LEIBINGER GMBH) 16.02.2006 zeigt ein chirurgisches Instrument zur Handhabung eines gebogenen chirurgischen Drahtes, mit zwei zur Erzeugung einer Klemmkraft relativ zueinander beweglichen Backen, wobei wenigstens eine der Backen eine Drahtaufnahme-Einrichtung aufweist.

US 5116340 A (SONGER ROBERT J (US); SONGER MATTHEW N (US)) 26.05.1992 zeigt ein zangenförmiges chirurgisches -Sicherungsinstrument mit zwei zur Erzeugung einer Klemmkraft relativ zueinander beweglichen Backen, wobei die Backen entgegengesetzte Aufnahmevorrichtungen zum Crimpen chirurgischer Drähte aufweist.

WO 96/39976 A (BURKE DENNIS W (US)) 19.12.1996 zeigt ein zangenförmiges Instrument zum Straffen und Crimpen von chirurgischem Draht in der orthopädischen Chirurgie, wobei der Draht in Aufnahmevorrichtungen der Klemmbacken geführt wird.

US 5314431 A (GRAZIANO THOMAS A) 24.05.1994 zeigt ein zangenförmiges Instrument zum Umbiegen von überstehendem chirurgischen Draht, dessen Klemmbacken eine gewinkelte Aufnahmevorrichtung für den chirurgischen Draht aufweisen.

DE 202005006132U (INTERCUS GMBH (DE)) 28.07.2005 zeigt ein Zielgerät von Kirschnerdrähten, wobei an einer Platte, an der ein Haltegriff angeordnet ist, wenigstens zwei Führungshülsen so zueinander angeordnet sind, dass sich die Achsen ausserhalb einer Fraktur kreuzen.

Aus der WO 01/35843 A (PIONEER LAB INC) 25.05.2001 ist eine Vorrichtung bekannt, die dazu geeignet ist, ein Kabel oder ein ähnliches Rundmaterial aufzunehmen und innerhalb der Vorrichtung zu klemmen. Dies wird dadurch bewirkt, dass ein Aufnahmeelement vorgesehen ist, das in Längserstreckung eine Bohrung aufweist. Diese Bohrung dient dazu, das Kabel, einen Draht oder ähnliches aufzunehmen. Ferner ist ein Klemmteil vorgesehen, das mit einem Griffteil gekoppelt ist. Durch Verschwenken des Griffteils wird bewirkt, dass ein Klemmelement, das Teil des Klemmteils ist, das Kabel innerhalb des Aufnahmeelements klemmt.

### Aufgabenstellung

Der Erfindung liegt die Aufgabe zugrunde, ein chirurgisches Instrument zur verbesserten Handhabung eines chirurgischen Drahtes anzubieten. Insbesondere soll das chirurgische Instrument dafür geeignet sein, um einen chirurgischen Draht in eine bereits vorgesehene Bohrung innerhalb eines Knochens fachgerecht einzuführen.

Diese Aufgabe wird gelöst durch ein chirurgisches Instrument entsprechend Anspruch 1.

### Vorteile der Erfindung

In einem ersten Schritt zur Drahtosteosynthese bei einer intraoperativen Frakturbehandlung, bei denen die Knochenfragmente mit Drähten, meistens so genannten Kirschnerdrähten oder K-Drähten, miteinander verbunden werden, ist es notwendig, das freie Ende des Drahts zunächst zu biegen. In der Regel sind Winkel zwischen 5 und 25 Grad vorgesehen, um ein Einführen in eine Bohrung, die innerhalb eines Knochens angebracht worden ist, zu ermöglichen. Alternativ ist auch vorgesehen, zunächst auf eine Biegung vollständig zu verzichten, so dass der zu implantierende chirurgische Draht vollständig gestreckt ist und derart in seiner Länge bemessen ist, dass er vollständig in dem erfindungsgemässen chirurgischen Instrument angeordnet ist. Damit ragt kein freies Ende des Drahtes aus dem Instrument heraus.

Die Erfindung geht somit den Weg, nicht ein zangenartig ausgebildetes chirurgisches Instrument zu verwenden, sondern zunächst ein Aufnahmeelement bereit zu stellen, das den zu applizierenden chirurgischen Draht vorzugsweise vollständig aufnimmt. Eine vollständige Aufnahme erfolgt in der Art, dass innerhalb des Aufnahmeelements zumindest in einem Teilbereich eine Ausnehmung vorhanden ist, in der ein Teil des langgestreckten chirurgischen Drahtes aufgenommen ist. Dadurch wird die Möglichkeit gegeben, dass innerhalb des Aufnahmeelements der zu applizierende chirurgische Draht gelagert ist, und zwar in der Art, dass dieser seine entsprechende Position innerhalb des Aufnahmeelements nicht verändern kann. Bei einer zangenartigen Ausbildung hingegen wäre der zu applizierende Draht letztendlich frei zwischen den Klemmbacken angeordnet, wobei beim Zudrücken des zangenartigen Instruments die Klemmbacken zumindest teilweise den zu applizierenden Draht umfassen. Der übrige Teil des chirurgischen Drahtes ist frei und offen zugänglich. Es besteht bei der Bedienung einer solchen Ausbildung eine erhebliche Verletzungsgefahr.

Der weitere wesentliche Vorteil der Erfindung besteht darin, dass relativ zu dem an sich feststehenden Aufnahmeelement, das auch Mittel bereitstellt, den zu applizierenden chirurgischen Draht aufzunehmen, ein beweglich gelagertes Klemmteil vorgesehen ist. Das Klemmteil ist derart ausgebildet, dass es ebenfalls einen Griffteil des gesamten Griffelements umfasst und auf dem dem Griffteil gegenüberliegenden Ende Klemmelemente vorgesehen sind, die zum Klemmen des Drahtes innerhalb des Aufnahmeelements ausgebildet sind.

Die Relation von Klemmteil und Aufnahmeelement ist derart ausgestaltet, dass durch Zusammendrücken der Griffteile mit einer Hand eine Klemmwirkung des chirurgischen Drahtes innerhalb des Aufnahmeelements erreicht wird, so dass ein Vorantreiben des Drahtes innerhalb der Bohrung ermöglicht wird. Wird zumindest ein Griffteil, sei es das des Klemmteils oder das des Aufnahmeelements wieder entlastet, so öffnet sich das Klemmteil und die Klemmwirkung reduziert sich. Dadurch ist es möglich, dass der Chirurg das chirurgische Instrument weiter von dem Knochen entfernt und der chirurgische Draht aber in seiner bereits eingeführten Position verharrt. Grund hierfür ist, dass die Haftreibung in der Bohrung in Bezug zu dem Draht grösser ist als die innerhalb des Aufnahmeelements. Dadurch wird wiederum mehr Draht aus dem chirurgischen Instrument freigegeben, so dass ein weiteres Implantieren des Drahtes innerhalb der Bohrung möglich ist.

Somit ist, wie gezeigt, ein wesentliches Merkmal dieser Erfindung, dass das chirurgische Instrument zum Applizieren eines chirurgischen Drahtes einhändig benutzt werden kann. Es genügt, dass das Aufnahmeelement in der Hand gehalten wird und das Klemmteil hierzu relativ mit den Fingern beziehungsweise der Hand auf- und zubewegt werden kann, so dass eine Klemmwirkung zum einen erreicht, zum anderen aber das Freigeben des Drahtes ebenfalls erzielt wird.

Eine Ausführungsform sieht vor, dass das erfindungsgemässe chirurgische Instrument aus zwei Bauteilen besteht, nämlich dem Aufnahmeelement zur Aufnahme des zu applizierenden Drahtes und dem Klemmteil zum Klemmen des Drahtes. Diese beiden Teile sind zangenartig miteinander gekoppelt. Das Aufnahmeelement weist Zapfen auf, die als Lager für die Anbindung des Klemmteils vorgesehen sind. Das Klemmteil ist derart gestaltet, dass es ösenartige Öffnungen aufweist, die dergestalt sind, dass das Klemmteil auf die Zapfen, die an dem Aufnahmeelement angeordnet sind, aufclipsbar ist und um diese Zapfen verschwenkbar ist.

Vorzugsweise sind Aufnahmeelement und Zapfen als einstückiges Teil ausgebildet, so dass es möglich ist, in einem einzigen Spritzgussverfahren das Aufnahmeelement und in einem weiteren Spritzgussverfahren das Klemmteil herzustellen.

Das Klemmteil selbst ist derart ausgebildet, dass in Bezug auf die Längserstreckung des Klemmteils der grössere Teil von einem Griffteil ausgebildet wird. Von dem Lager sich weg erstreckend (weg von dem Griffteil) ist die Klemmfläche angeordnet, die vorzugsweise eine entsprechende Ausnehmung aufweist, die auf den Durchmesser des zu applizierenden chirurgischen Drahtes angepasst ist. Klemmteil und Klemmfläche bilden vorzugsweise ein einstückiges Teil, so dass auch hier ein Herstellen in einem einzigen Spritzgussverfahren möglich ist.

Eine Weiterbildung sieht vor, dass die Klemmflächen des Klemmteils mit einem Metallelement besetzt sind. Entweder kann das Metallelement, welches die Klemmfläche umgibt, nachträglich aufgebracht werden oder es wird bereits in dem besagten Spritzgussverfahren mit eingearbeitet. Durch dieses Metallelement kann die Klemmkraft weiter erhöht und der in diesem Bereich auftretende Verschleiss reduziert werden.

Eine Weiterbildung sieht vor, dass die Klemmfläche profiliert ist. Dies bedeutet, dass die Klemmfläche geriffelt sein kann, mit der Zielsetzung die Reibkraft zwischen dem zu klemmenden Draht und der Klemmfläche zu erhöhen. Alternativ oder ergänzend kann vorgesehen sein, ein Profil, beispielsweise eine Kerbe in Richtung der Längserstreckung des Drahtes vorzusehen, so dass sich der zu klemmende Draht während des Klemmvorgangs in die Kerbe hineinarbeitet und dort zusätzlich geklemmt wird. Die Kerben können unterschiedliche Ausgestaltungen haben.

Eine weitere vorteilhafte Ausbildungsform sieht auch vor, dass im Bereich der Ausnehmung im Aufnahmeelement ebenfalls zusätzliche Klemm- oder Führungsmittel vorgesehen sind, die dazu geeignet sind, den zu applizierenden chirurgischen Draht aufzunehmen. Dabei ist die Ausnehmung, beispielsweise in Form einer Kehle, derart ausgebildet, dass sie chirurgische Drähte in unterschiedlichen Durchmessern aufnehmen kann.

Diese beschriebenen Profile haben auch den Vorteil, dass durch diese Massnahme der zu applizierende Draht innerhalb des Aufnahmeelements genau positioniert wird, so dass - bei auf das freie Ende des Drahts wirkenden äusseren Kräfte - kein Verschieben des Drahtes innerhalb des Instruments stattfindet.

Somit ist gewährleistet, dass das erfindungsgemässe chirurgische Instrument dazu geeignet ist, chirurgische Drähte, beispielsweise Kirschnerdrähte von einem geringen Durchmesser, beispielsweise 0,8 mm bis zu 2 mm in einem einzigen Gerät aufzunehmen und zu applizieren. Die Erfindung ist jedoch nicht nur auf die Aufnahme dieser angegebenen Durchmesser beschränkt. Vielmehr erstreckt sich der Schutzbereich in Bezug auf Drähte auf solche Grössen und Querschnitte, die zum Fixieren von Knochen Anwendung finden. Dafür sind viele unterschiedliche Durchmesser aber auch unterschiedliche Querschnitte geeignet.

Eine Weiterbildung der Erfindung sieht vor, dass in dem Aufnahmeelement auch Biegevorrichtungen vorgesehen sind. Diese Biegevorrichtungen sind derart gestaltet, dass beispielsweise ein freies Ende eines chirurgischen Drahtes entsprechend gebogen werden kann, so dass der gebogene Teil zum übrigen sich längserstreckenden Teil einen definierten Winkel einnimmt. Eine ebenfalls in dem Aufnahmeelement vorgesehene Kontrolleinrichtung in der Ausbildung einer Lehre kann zur Kontrolle und Abmessung des erzeugten Winkels herangezogen werden.

Eine bevorzugte Ausbildung des Instruments sieht vor, dass das gesamte Instrument aus Metall hergestellt ist. Aufgrund dieser Materialwahl ist es sterilisierbar.

Eine andere Ausführungsform sieht vor, dass das Produkt aus Kunststoff gefertigt ist. Damit kann es als ein Einmal-Produkt eingesetzt werden. Um eine gute Klemmkraft zu erreichen, ist der Klemmbereich bei einer Kunststoffausführung metallisiert beziehungsweise mit einer Metallplatte versehen.

An dem freien Ende des Griffelements ist eine Hilfsvorrichtung vorgesehen, die die Eigenschaft aufweist, dass die beiden Klemmteile nur dann zusammengedrückt werden können, wenn der chirurgische Draht nicht auf der Griffseite über das freie Ende des chirurgischen Instruments hinaussteht. Eine laschenartige Ausbildung, die sich von dem Klemmteil in Richtung des freien Endes des Aufnahmeelements erstreckt, ist derart ausgebildet, dass im zusammengedrückten Zustand des chirurgischen Instrumentes diese freie Lasche auch das freie Ende des Aufnahmeelements überdeckt. Damit wird auch das freie Ende der Bohrung, in der der Draht für die Applikation geführt wird, abgedeckt und damit vermieden, dass der Draht auf der Griffseite ungewollt austritt. Dadurch ist ein Überstehen des chirurgischen Drahtes über die Griffseite hinweg nicht möglich und eine Verletzung an der Hand des Chirurgen damit ausgeschlossen.

### Ausführungsbeispiel

### Kurze Beschreibung der Zeichnungen

Weitere Einzelheiten und Vorteile der Erfindung ergeben sich aus der nachfolgenden Beschreibung eines bevorzugten Ausführungsbeispiels der Erfindung sowie aus den Figuren.
Es zeigen:

Fig. 1 eine perspektivische Ansicht auf ein erfindungsgemässes chirurgisches Instrument;

Fig. 2 eine perspektivische Ansicht auf ein erfindungsgemässes chirurgisches Instrument;

Fig. 3 eine schematische Darstellung des Klemmprinzips des chirurgischen Instruments gemäss den Fig. 1 und 2;

Fig. 4 einen Schnitt durch das chirurgische Instrument gemäss Fig. 1 oder 2, wobei gegenüber Fig. 1 oder 2 ein chirurgischer Draht aufgenommen ist, der jedoch nicht geklemmt ist;

Fig. 5 einen Schnitt durch das chirurgische Instrument gemäss Fig. 1 oder 2, wobei gegenüber Fig. 1 ein chirurgischer Draht aufgenommen ist, der geklemmt ist;

Fig. 6 a und b eine schematische Darstellung eines Schnittes durch das chirurgische Instrument gemäss Fig. 5 entlang einer Linie VI-VI zur Darstellung der Klemmwirkung unterschiedlicher Durchmesser von chirurgischen Drähten;

Fig. 7 a und b eine Darstellung der Funktionsweise der in dem Aufnahmeelement vorgesehenen Biegeeinrichtung;

Fig. 8 eine schematische Darstellung der Anwendung des chirurgischen Instruments gemäss den vorstehenden Figuren.

### Beschreibung eines bevorzugten Ausführungsbeispiels

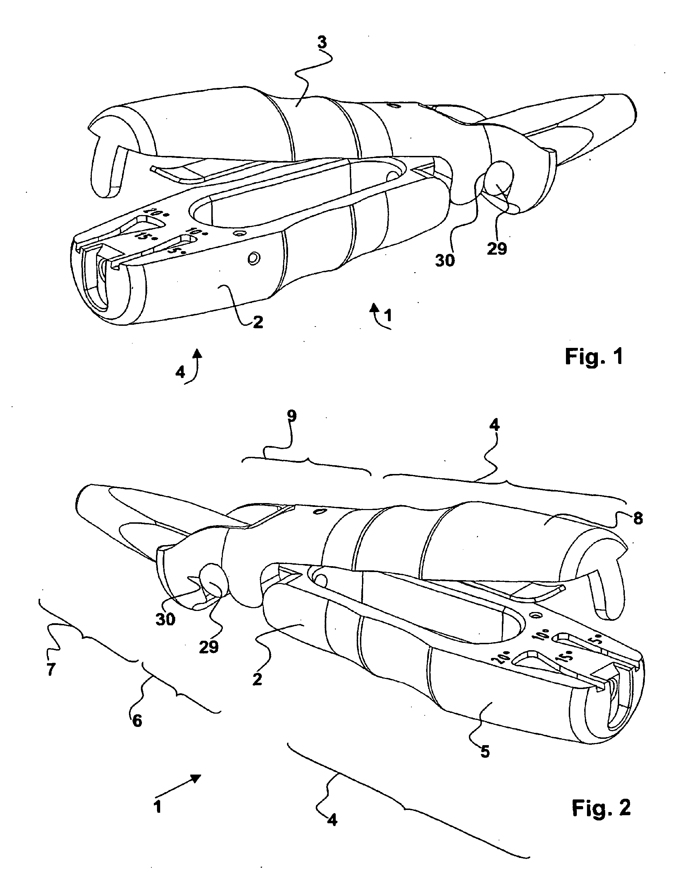

Nachfolgend wird unter Bezugnahme auf die Figuren 1-8 ein Ausführungsbeispiel eines erfindungsgemässen chirurgischen Instruments beispielhaft beschrieben.

Fig. 1 und 2 zeigen eine perspektivische Gesamtansicht des erfindungsgemässen chirurgischen Instruments 1 zur Handhabung und Implementierung eines chirurgischen Drahts, vorzugsweise eines Kirschnerdrahts. Der Draht ist bei dem hier dargestellten Ausführungsbeispiel in den Fig. 1 und 2 nicht gezeigt.

Das chirurgische Instrument 1 weist zwei Bauteile auf, nämlich ein Aufnahmeelement 2 und ein Klemmteil 3. Das chirurgische Instrument selbst umfasst ein Griffelement 4, mit dem das chirurgische Instrument über eine Hand bedient werden kann.

Das Aufnahmeelement 2 weist im Wesentlichen drei Funktionsbereiche auf. Der erste Funktionsbereich ist derjenige des Griffelements 4 und wird durch ein Griffteil 5 gebildet. An das Griffteil 5 sich anschliessend ist eine Lagerstelle 6 vorgesehen, wobei an die Lagerstelle 6 sich wiederum ein Führungs- und Positionierelement 7 anschliesst. Die Lagerstelle 6 umfasst Zapfen 29, die mit einem Lagerelement 9 des Klemmteils 2 zusammenwirken.

Das Klemmteil 3 weist ebenfalls drei Funktionsbereiche auf. Der erste Funktionsbereich ist derjenige des Griffelements 4 und wird durch ein Griffteil 8 gebildet. An das Griffteil 8 schliesst sich wiederum ein Lagerelement 9 an, das mit der Lagerstelle 6 des Aufnahmeelements 2 zusammenwirkt. An das Lagerelement 9 sich anschliessend ist ein das Klemmteil umfassende Klemmelement 10 vorgesehen.

Beide Bauteile, nämlich das Aufnahmeelement 2 und das Klemmteil 3 sind als einstückige Teile ausgebildet und bilden durch Zusammenwirken über die Lagerstelle 6 beziehungsweise das Lagerelement 9 das chirurgische Instrument 1.

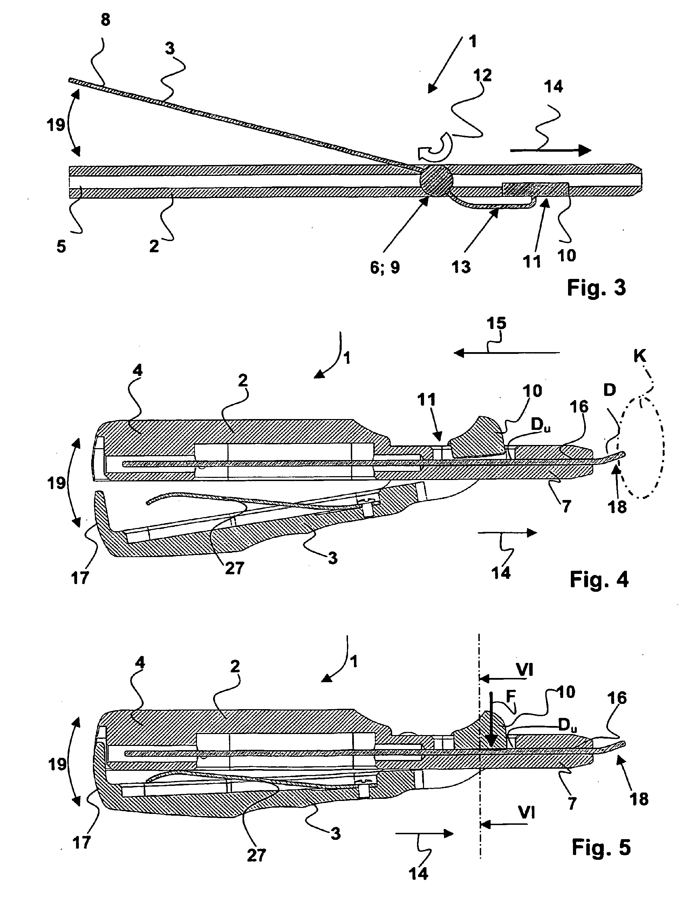

In Fig. 3 ist das Prinzip des erfindungsgemässen chirurgischen Instruments 1 dargestellt. In dem Aufnahmeelement 2 ist der zu applizierende Draht gelagert. Das Klemmteil 3 ist über Lagerstellen 6, 9 mit dem Aufnahmeelement gekoppelt. Das Klemmteil 3 weist an seiner dem Griffteil 8 abweisenden Seite das Klemmelement 10 auf, das durch Verschwenken des Klemmteils 3 in Pfeilrichtung 12 eine Klemmung in Pfeilrichtung 13 vollzieht. Dabei durchdringt das Klemmelement 10 eine in dem Aufnahmeelement 2 vorgesehene Ausnehmung 11. Durch die Klemmung ist der Draht in und gegen die Pfeilrichtung 14 fixiert. In dieser Situation, die dadurch erreicht wird, dass die beiden Griffteile 5, 8 in Pfeilrichtung 19 zusammengedrückt werden, kann der Draht appliziert werden.

### Funktionsweise

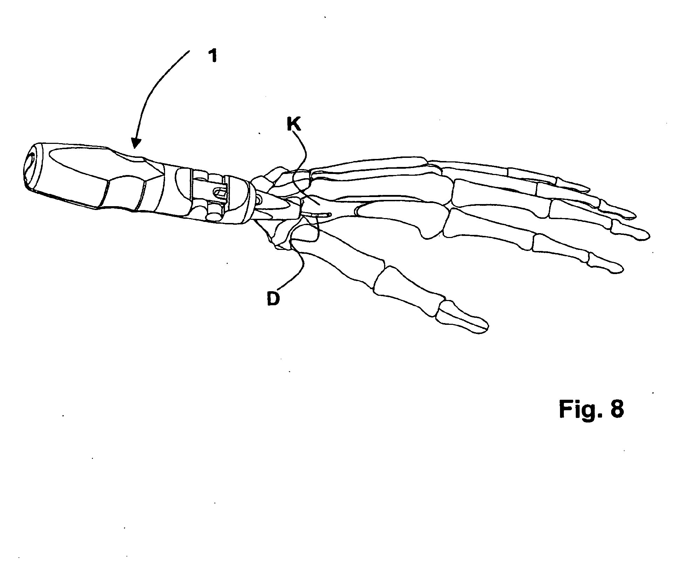

In den Fig. 4 und 8 ist die Funktionsweise des chirurgischen Instruments 1 dargestellt.

Fig. 4 zeigt, dass der Draht D in das Aufnahmeelement 2 eingefügt worden ist. Der Draht D wird beim Einführen in das chirurgische Instrument 1 in Pfeilrichtung 15 über das Führungs- und Positionierelement 7, das eine Bohrung 16 aufweist, eingeführt und bis an einen Anschlag 17, der im Bereich des Griffelements 4 vorgesehen ist, geschoben. Damit der Draht D an den Anschlag 17 fährt, wird das Griffelement 4 leicht zusammengedrückt, so dass noch keine Klemmung stattfindet.

Eine Alternative zur Einführung des Drahts D besteht darin, dass dieser rückwärtig, d.h. entgegengesetzt zu dem Führungs- und Positionierungselement 7 eingeführt wird, bis das eine Ende des Drahts D vollständig innerhalb des Aufnahmeelements 2 positioniert ist und das Griffelement 4 zusammendrückbar ist.

Je nach Anwendungsfall kann der zu applizierende chirurgische Draht auch in seinem Anfangsbereich 18 gebogen ausgestaltet sein, so wie es in Fig. 4 exemplarisch dargestellt ist. Zum Einführen des chirurgischen Drahts D in das Aufnahmeelement 2 ist es notwendig, dass die beiden Griffteile 4 und 5 derart voneinander beabstandet sind, dass das Klemmelement 10 nicht auf den Aussenumfang Dᵤ zur Anlage gelangt. In dieser Situation tritt keine Klemmwirkung, hervorgerufen durch das Klemmteil 3, ein.

Für das Applizieren des chirurgischen Drahtes D muss die in Fig. 5 dargestellte Situation erreicht werden. Diese wird dadurch erreicht, dass die beiden Griffteile 5, 8 in Pfeilrichtung 19 zusammengedrückt werden, so dass das Klemmelement 10 unmittelbar auf den Aussenumfang Dᵤ des Drahts D drückt. Durch das Drücken entsteht eine Kraftwirkung F senkrecht zur Längserstreckung des Drahts D und der Draht D wird somit gegen die Innenwandung der Bohrung 16 des Aufnahmeelements 2 gedrückt und dort auch geklemmt, so dass ein Verschieben des Drahts D in und gegen Pfeilrichtung 14 nicht mehr möglich ist. Mit dieser Klemmeinstellung kann der Draht D appliziert werden. Gelangt das vordere freie Ende des Führungs- und Positionierelements 7 in den unmittelbaren Bereich der in Fig. 4 nur strichpunktiert angedeuteten Wandung des Knochens K, so hat der Operateur die beiden Griffteile 5 und 8 zu entlasten, so dass die in Fig. 4 dargestellte Situation erreicht wird. Aufgrund der Gegenkraft, die beim Zusammendrücken der beiden Griffteile entsteht, erreicht der Operateur die in Fig. 4 dargestellte Situation nur dadurch, dass er seine Handflächen etwas weiter öffnet, so dass die beiden Griffteile 5, 8 selbsttätig entgegen der Pfeilrichtung 19 aufspringen können. Dabei ist es nicht notwendig, dass der Operateur das Griffelement 4 vollständig los lässt, sondern es reicht aus, die Finger ausschliesslich geringfügig zu öffnen und das chirurgische Instrument 1 entgegen der Pfeilrichtung 14 weg von dem Knochen K zu ziehen. Ist der Abstand zur Aussenwandung des Knochens K wieder ausreichend gross, so kann der Operateur wieder die gewünschte Klemmwirkung herbeiführen und den Draht D weiter applizieren. Diesen Vorgang wiederholt der Operateur so lange, bis der Draht D vollständig appliziert ist.

Das Aufspringen der beiden Griffteile 5 und 8 wird bei einem besonders bevorzugten Ausführungsbeispiel durch ein Federelement 27, wie es in den Fig. 1, 2, 4 und 5 dargestellt ist, unterstützt. Durch Zudrücken der beiden Grittteile 5 und 8 in Pfeilrichtung 19 wird in dem Federelement Energie gespeichert, die dann beim Loslassen der Griffteile 5 und 8 freigegeben wird.

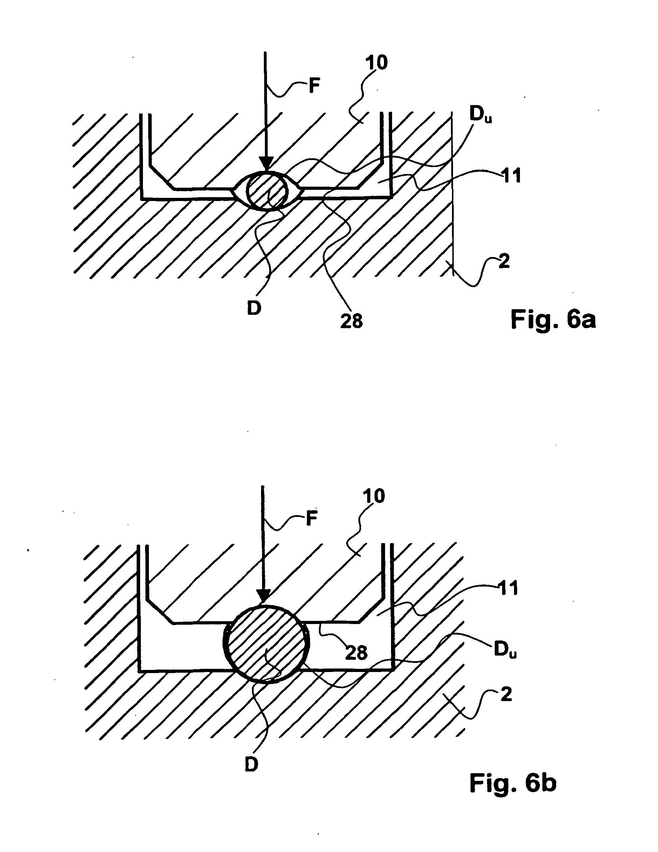

In Fig. 6 a und b ist schematisch ein Schnitt durch das in Fig. 5 dargestellte chirurgische Instrument 1 gezeigt. Dabei ist eine Klemmwirkung dargestellt, die dadurch hervorgerufen wird, dass das Klemmelement 10 gegen den Aussenumfang Dᵤ des Drahtes D drückt und somit den Draht D gegen das Aufnahmeelement 2 presst. Dabei wirkt eine Klemmkraft F, wie sie in Fig. 6 a und b dargestellt ist.

Die Klemmteile 10 und auch ein Teil der Ausnehmung 11 sind derart gestaltet, dass unterschiedliche Durchmesser von chirurgischen Drähten D aufgenommen werden können. In Fig. 6a ist dargestellt, wie ein chirurgischer Draht D mit einem geringen Durchmesser aufgenommen und eine entsprechende Klemmkraft F erzeugt werden kann. In Fig. 6b ist ein chirurgischer Draht D dargestellt, bei dem der Durchmesser des Drahts D erheblich grösser ist. Die jeweils dargestellten Klemmflächen 28 des Klemmelements 10 können dabei unterschiedlich gestaltete Profile aufweisen. Unabhängig von dem entsprechenden Durchmesser des jeweiligen Drahtes D kann die erforderliche Klemmkraft F trotzdem dargestellt werden. Alle Profile haben die Eigenschaft, dass die Reibung und/oder die Klemmkraft zwischen dem Draht und dem Klemmelement 10 erhöht wird. Somit ist ein Applizieren von unterschiedlichen Drähten mit unterschiedlichen Durchmessern mit ein und demselben chirurgischen Instrument 1 möglich.

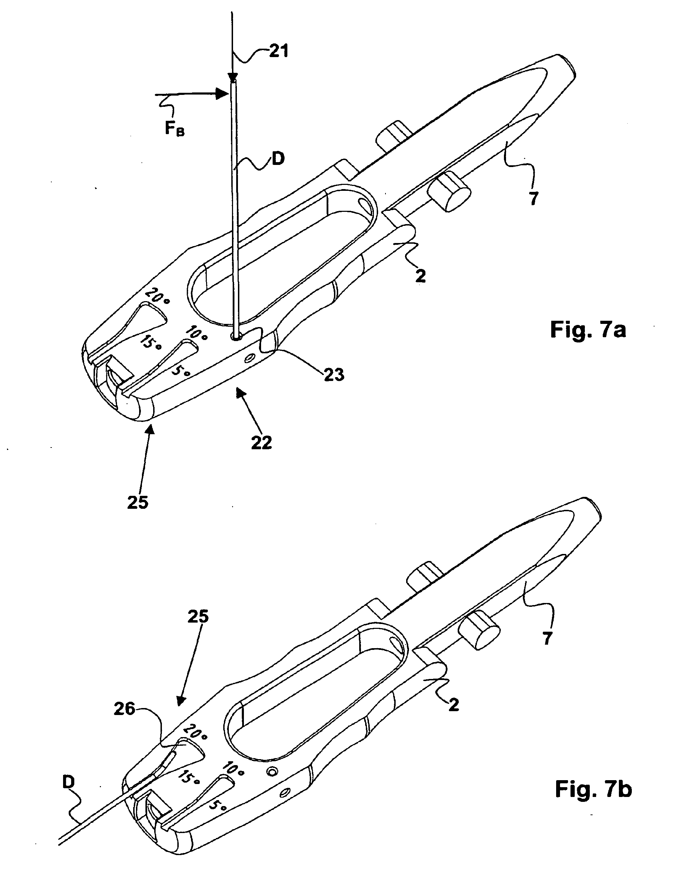

Das Aufnahmeelement 2 weist bei einem weiteren bevorzugten Ausführungsbeispiel eine Einrichtung 22 auf (Fig. 7a). Diese umfasst eine Bohrung 23, in die der zu bearbeitende Draht D in Pfeilrichtung 24 eingefügt wird. Sobald die entsprechende Position erreicht ist, wirkt eine Kraft F_{B} an den Aussenumfang des Drahtes und führt so die Biegung herbei. Insbesondere dient diese Einrichtung 22 dazu, das freie Ende des chirurgischen Drahtes D zu biegen, um das Einführen in eine Bohrung innerhalb eines Knochens zu erleichtern.

Um zu prüfen, ob der gewollte Biegewinkel des freien Ende des Drahts D auch erreicht ist, ist, so wie in den Fig. 7a und b dargestellt, ein Kontrollbereich 25 vorgesehen. Dieser Kontrollbereich 25 zeichnet sich dadurch aus, dass dieser unterschiedliche Segmente für die Kontrolle des Biegewinkels aufweist. So ist beispielsweise eine Kontrolllehre 26 dargestellt, bei der der Draht D, so wie in Fig. 7b dargestellt, in die Kontrollehre 26 eingelegt wird. Liegt der Aussenumfang des Drahtes D an der Kontur der Kontrollehre 26 an, so ist der definierte Winkel, hier 20 Grad, erreicht. Weitere Kontrollehren können vorgesehen werden.

Das erfindungsgemässe chirurgische Instrument 1 hat somit viele Vorzüge. Einer der wesentlichen Vorzüge ist, dass es aufgrund des Griffelements 4 und dessen Ausgestaltung einhändig bedienbar ist. Das Griffelement 4 wird damit zum verlängerten Arm des Chirurgen.

Aufgrund der Anordnung der Lagerstelle 6 beziehungsweise Lagerelemente 9 des Klemmteils 3 in Relation zum Aufnahmeelement 2 können erhebliche Klemmkräfte erzielt werden, da die Hebelwirkung sich vervielfacht.

Das chirurgische Instrument ist leicht zerlegbar und somit gut zu reinigen und auch entsprechend zu sterilisieren.

Die weiteren Ausführungsformen weisen bevorzugte Biegeeinrichtungen auf, mittels der das freie Ende des Drahtes entsprechend mit einem Einführungswinkel versehen werden kann. Ein und dasselbe chirurgische Instrument ist für die Aufnahme von chirurgischen Drähten, insbesondere Kirschnerdrähten, geeignet.

### Bezugszeichenliste

1. Chirurgisches Instrument
2. Aufnahmeelement
3. Klemmteil
4. Griffelement
5. Griffteil
6. Lagerstelle
7. Führungs- und Positionierungselement
8. Griffteil
9. Lagerelement
10. Klemmelement
11. Ausnehmung
12. Pfeilrichtung
13. Pfeilrichtung
14. Pfeilrichtung
15. Pfeilrichtung
16. Bohrung
17. Anschlag
18. Anfangsbereich
19. Pfeilrichtung
20. ---
21. Pfeilrichtung
22. Biegeeinrichtung
23. Bohrung
24. Pfeilrichtung
25. Kontrollbereich
26. Kontrolllehre
27. Federelement
28. Klemmfläche
29. Zapfen
30. Ausnehmung
D Draht
Du Aussenumfang des Drahtes D
F Kraftwirkung (Klemmkraft)
K Knochen
FB Biegekraft

## Patentansprüche

1. Chirurgisches Instrument (1) zur Implantierung eines Drahtes (D), vorzugsweise in einen Knochen (K), bestehend im Wesentlichen aus
- einem Aufnahmeelement (2) zumindest zur Aufnahme des zu applizierenden Drahtes (D), wobei das Aufnahmeelement (2) eine Ausnehmung (11) zur zumindest teilweisen Aufnahme des Drahtes (D) umfasst,
- einem Klemmteil (3) zumindest zum Klemmen des Drahts (D), wobei das Klemmteil (3) drehbeweglich über eine Lagerstelle (6) an dem Aufnahmeelement (2) gelagert ist und ein Klemmelement (10) umfasst,
- einem Griffelement (4) zum Halten des chirurgischen Instruments (1) und zum Klemmen und Freigeben des in der Ausnehmung (11) vorsehbaren Drahtes (D)
**dadurch gekennzeichnet, dass**
- das Griffelement (4) aus zwei Griffteiten (5, 8) besteht, wobei das Aufnahmeelement (2) das erste Griffteil (5) sowie das Klemmteil (3) das zweite Griffteil (8) umfasst,
- das Klemmteil (3) und das Aufnahmeelement (2) zangenartig zerlegbar miteinander gekoppelt sind, indem hierfür das Aufnahmeelement (2) Zapfen (29) und das Klemmteil (3) ösenartige Lagerelemente (9) mit zu einer Seite vorgesehenen Öffnungen (30) aufweisen, und zwar derart, dass das Klemmteil (3) auf die Zapfen (29) aufclipbar und um diese schwenkbar ist,
- das Klemmelement (10) des Klemmteils (3) dabei in die Ausnehmung (11) des Aufnahmeelements (2) eingreift und derart ausgestaltet ist, dass durch ein Zusammendrücken der Griffteile (5, 8) der Draht (D) durch das Klemmelement (10) gegen das Aufnahmeelement (2) drücktbar ist.

2. Chirurgisches Instrument nach Anspruch 1, **dadurch gekennzeichnet, dass** die Zapfen (29) einstückig mit dem Aufnahmeelement (2) verbunden sind.

3. Chirurgisches Instrument nach Anspruch 1 oder 2, **dadurch gekennzeichnet, dass** die Lagerelemente (9) einteilig mit dem Klemmteil (3) ausgebildet sind.

4. Chirurgisches Instrument nach mindestens einem der vorhergehenden Ansprüche, **dadurch gekennzeichnet, dass** das Aufnahmeelement (2) ein Führungs- und Positionselement (7) umfasst, das sich an die Largestelle (6) anschließt und eine innen liegende Bohrung (16) zur Aufnahme des Drahts (D) umfasst.

5. Chirurgisches Instrument nach mindestens einem der vorhergehenden Ansprüche, **dadurch gekennzeichnet, dass** das Aufnahmeelement (2) eine Biegeeinrichtung (22) für das freie Ende des Drahtes (D) umfasst, die durch eine Bohrung (23) in diesem gebildet ist.

6. Chirurgisches Instrument nach Anspruch 5, **dadurch gekennzeichnet, dass** die Biegeeinrichtung (22) mindestens eine in dem Aufnahmeelement (2) vorgesehene Kontrolllehre (26) zur Kontrolle eines Biegewinkels am Draht (D) umfasst, der mit der Biegeeinrichtung (22) erhaltbar ist

## Claims

1. Surgical instrument (1) for implanting a wire (D), preferably in a bone (K), consisting principally of
- a receiving element (2) at least for receiving the wire (D) to be applied, wherein the receiving element (2) comprises a recess (11) for at least partially receiving the wire (D),
- a clamp part (3) at least for clamping the wire (D), wherein the clamp part (3) is mounted on the receiving element (2) so as to be able to move in rotation about a bearing point (6) and comprises a clamp element (10),
- a grip element (4) for holding the surgical instrument (1) and for clamping and releasing the wire (D) that can be provided in the recess (11),
**characterized in that**
- the grip element (4) consists of two grip parts (5, 8), wherein the receiving element (2) comprises the first grip part (5), and the clamp part (3) comprises the second grip part (8),
- the clamp part (3) and the receiving element (2) are coupled detachably to each other like pliers, for which purpose the receiving element (2) has pins (29) and the clamp part (3) has eyelet-like bearing elements (9) with openings (30) provided to one side, specifically in such a way that the clamp part (3) can be clipped onto the pins (29) and is pivotable about the latter,
- the clamp element (10) of the clamp part (3) engages here in the recess (11) of the receiving element (2) and is designed in such a way that, when the grip parts (5, 8) are pressed together, the clamp element (10) presses the wire (D) against the receiving element (2).

2. Surgical instrument according to Claim 1, **characterized in that** the pins (29) are connected integrally to the receiving element (2).

3. Surgical instrument according to Claim 1 or 2, **characterized in that** the bearing elements (9) are designed in one piece with the clamp part (3).

4. Surgical instrument according to at least one of the preceding claims, **characterized in that** the receiving element (2) comprises a guiding and positioning element (7), which adjoins the bearing point (6) and comprises an inner bore (16) for receiving the wire (D).

5. Surgical instrument according to at least one of the preceding claims, **characterized in that** the receiving element (2) comprises a bending device (22) for the free end of the wire (D), which bending device (22) is formed by a bore (23) therein.

6. Surgical instrument according to Claim 5, **characterized in that** the bending device (22) comprises at least one control gauge (26) provided in the receiving element (2) for controlling a bending angle on the wire (D), which bending angle can be obtained with the bending device (22).

## Revendications

1. Instrument chirurgical (1) destiné à l'implantation d'un fil (D), de préférence dans un os (K), constitué essentiellement
- d'un élément de réception (2) destiné au moins à recevoir le fil (D) à appliquer, l'élément de réception (2) comprenant un évidement (11) destiné à recevoir au moins partiellement le fil (D),
- d'une partie de serrage (3) destinée au moins à serrer le fil (D), la partie de serrage (3) étant montée de manière mobile en rotation par le biais d'un point de palier (6) sur l'élément de réception (2) et comprenant un élément de serrage (10),
- d'un élément de préhension (4) destiné à retenir l'instrument chirurgical (1) et à serrer et libérer le fil (D) pouvant être prévu dans l'évidement (11),
**caractérisé en ce que**
- l'élément de préhension (4) se compose de deux parties de préhension (5, 8), l'élément de réception (2) comprenant la première partie de préhension (5) et la partie de serrage (3) comprenant la deuxième partie de préhension (8),
- la partie de serrage (3) et l'élément de réception (2) sont accouplés l'un à l'autre à la manière d'une pince de manière séparable, l'élément de réception (2) présentant à cet effet des tourillons (29) et la partie de serrage (3) présentant à cet effet des éléments de palier de type oeillet (9) avec des ouvertures (30) prévues d'un côté, et ce de telle sorte que la partie de serrage (3) puisse être enclipsée sur les tourillons (29) et puisse pivoter autour de ceux-ci,
- l'élément de serrage (10) de la partie de serrage (3) vient en prise en l'occurrence dans l'évidement (11) de l'élément de réception (2) et est configuré de telle sorte que par une compression des parties de préhension (5, 8), le fil (D) puisse être pressé par l'élément de serrage (10) contre l'élément de réception (2).

2. Instrument chirurgical selon la revendication 1, **caractérisé en ce que** les tourillons (29) sont connectés d'une seule pièce à l'élément de réception (2).

3. Instrument chirurgical selon la revendication 1 ou 2, **caractérisé en ce que** les éléments de palier (9) sont réalisés d'une seule pièce avec la partie de serrage (3).

4. Instrument chirurgical selon au moins l'une quelconque des revendications précédentes, **caractérisé en ce que** l'élément de réception (2) comprend un élément de guidage et de positionnement (7) qui se raccorde au point de palier (6) et qui comprend un alésage (16) situé à l'intérieur pour recevoir le fil (D).

5. Instrument chirurgical selon au moins l'une quelconque des revendications précédentes, **caractérisé en ce que** l'élément de réception (2) comprend un dispositif de flexion (22) pour l'extrémité libre du fil (D), qui est formé à travers un alésage (23) dans celui-ci.

6. Instrument chirurgical selon la revendication 5, **caractérisé en ce que** le dispositif de flexion (22) comprend au moins un guide de contrôle (26) prévu dans l'élément de réception (2) pour contrôler un angle de flexion au niveau du fil (D), lequel peut être obtenu avec le dispositif de flexion (22).
